# EUROPÄISCHE PATENTANMELDUNG

(11) **EP 1 060 685 A2**
(43) Veröffentlichungstag der Anmeldung: **20.12.2000**
(21) Anmeldenummer: 00105785.0
(22) Anmeldetag: 18.03.2000
(51) Int. Cl.: A45D 34/02

(54) **Verfahren zur Vermittlung eines bestimmten Geruchseindrucks auf eine Person sowie eine Vorrichtung zur Durchführung des Verfahrens**

(30) Priorität: 11.06.1999 DE 19926795; 09.07.1999 DE 19932107
(71) Anmelder: Wella Aktiengesellschaft, 64274 Darmstadt (DE)
(72) Erfinder: Ude, Christiane, 64342 Seeheim-Jugenheim (DE)

(57) **Zusammenfassung**

Verfahren zur Vermittlung eines bestimmten Geruchseindrucks auf eine Person (2) mittels eines Duftstoffpräparats (3), wobei ein gebündelter Luftstrom (5) wenigstens ungefähr auf einen Ansaugbereich (7) einer Nase (8) der Person (2) gerichtet wird und das Duftstoffpräparat (3) vom Luftstrom (5) umspült oder/und durchspült wird. Als Vorrichtung (1) zur Vermittlung eines bestimmtem Geruchseindrucks auf eine Person (2) mittels eines Duftstoffpräparats (3) ist die Vorrichtung (1) mit einer Einrichtung (4) zur Erzeugung mindestens eines gebündelten Luftstromes (5) versehen, wobei das Duftstoffpräparat (3) innerhalb des Luftstromes (5) angeordnet ist und vom Luftstrom (5) umspült oder/und durchspült wird, und daß der so mit einem Duft (6) versehene Luftstrom (5) wenigstens ungefähr auf einen Ansaugbereich (7) einer Nase (8) der Person (2) gerichtet ist.

## Beschreibung

Die Erfindung betrifft ein Verfahren nach der Gattung des Oberbegriffs des Anspruchs 1 sowie eine Vorrichtung nach der Gattung des Oberbegriffs des Anspruchs 2.

Gängige Methoden zur Erzeugung von Duft zielen darauf ab, einen ganzen Raum mit Duft auszufüllen. Dies hat zur Folge, daß ein Wechsel der Geruchsrichtung kaum möglich ist, da es zu einer Anreicherung der Duftstoffe in dem Raum und dadurch zu unangenehmen Mischgerüchen kommt. Auch ist es damit nicht möglich, einzelnen Personen eine angenehme Geruchsempfindung zu verschaffen, ohne daß andere Personen diesen Gerüchen auch ausgesetzt werden. Es ist zwar aus der DE 38 43 871 A1 bekannt, einen bestimmten Geruchseindruck für nur eine einzelne Person zu vermitteln, was aber durch den Nachteil erreicht wird, daß ein Duftpräparat mittels eines Bügels im Nasenbereich einer Person angeordnet ist.

Es ist daher Aufgabe der Erfindung, ein Verfahren bzw. eine Vorrichtung nach der Gattung des Oberbegriffs des Anspruchs 1 bzw. des Anspruchs 2 zu schaffen, das bzw. die die oben genannten Nachteile nicht aufweist.

Gelöst wird diese Aufgabe nach den Merkmalen des kennzeichnenden Teil des Anspruchs 1 bzw. des Anspruchs 2. Vorteilhafte Weiterbildungen gehen aus den Unteransprüchen hervor.

Dadurch, daß der Luftstrom ungefähr eine Raumtemperatur aufweist, wird ein veränderter Geruchseindruck durch Wärmeeinfluß verhindert (Anspruch 3).

Dadurch, daß die Einrichtung mit mindestens einem elektrischen Gebläse versehen ist, läßt sich einfach ein Luftstrom erzeugen und richten (Anspruch 4).

Dem Gebläse ist ein Luftansaugteil, ein Luftströmungsgleichrichter und das Duftstoffpräparat zugeordnet, wodurch sich eine kompakte Einheit ergibt (Anspruch 5).

Eine vorteilhafte Anwendung der Vorrichtung ergibt sich dadurch, daß die Vorrichtung mit einem Gerät zum Behandeln von Kopfhaar verbunden ist (Anspruch 6).

Eine gute Handhabung und Verbindungsmöglichkeit mit der Vorrichtung ergibt sich dadurch, daß das Duftstoffpräparat mit einer Halteeinrichtung versehen ist (Anspruch 7).

Eine einfache Handhabung und Kostenvorteile des Duftstoffpräparats ergeben sich dadurch, daß das Duftstoffpräparat als eine Karte (billiges Stanzteil) ausgestaltet ist (Anspruch 8).

Vorteilhafterweise ist das Duftstoffpräparat von einem saugfähigem Material aufgenommen, wodurch durch einen Kapillareffekt weitgehend eine gleichmäßige Duftabgabe ermöglicht ist (Anspruch 9).

Ein sehr kostengünstiges Duftstoffpräparat ergibt sich dadurch, daß das saugfähige Material als eine Karte, wahlweise steckbar, ausgestaltet ist, wobei das Duftstoffpräparat zumindest von einem Teil des saugfähigen Materials aufgenommen ist (Anspruch 10).

Eine Konservierung des Duftstoffes des Duftstoffpräparats wird dadurch erreicht, daß die Karte mit einer Duftschutz-Umhüllung versehen ist (Anspruch 11).

Dadurch, daß das saugfähige Material des Duftstoffpräparats als ein Zylinder ausgestaltet ist, läßt es sich mit einer Umhüllungskappe als Duftschutz verschließen und ist daher auch für eine Duftprobe geeignet (Ansprüche 12 und 13).

Durch ein System mit unterschiedlichen Duftstoffarten ist eine individuelle Vermittlung eines bestimmten Geruchseindrucks durch Auswahl möglich (Anspruch 14).

Aus Gründen von Keimfreiheit ist das Duftstoffpräparat mit der Halteeinrichtung als Einwegteil ausgestaltet (Anspruch 15).

Die Karten sind mit einer Information über die Duftstoffart versehen, beispielsweise mittels einer bestimmten Farbgebung oder/und eines bestimmten Symbols oder/und eines Beschreibungsaufdrucks (Ansprüche 16 und 17).

Durch einen Luftstromformungsvorsatz an der Vorrichtung wird die Luftströmung so aufbereitet, daß für einen Riecheffekt bezüglich eines Einsaugens von Duftstoffen mittels einer Nase 8 ideale Bedingungen durch feine Verwirbelungen und Bremsung der Luftströmung vorliegen, so daß eine Konzentration von Duftstoffen sehr niedrig sein kann. Dadurch wird keine Inhalation von Duftstoffen ausgelöst, sondern gerade die Schwelle des Geruchsempfindens erreicht (Anspruch 18).

Alternativ zum Gebläse kann der Luftstrom auch von Druckluft oder Dampf gebildet werden (Anspruch 19).

Mittels einer Programmsteuerung kann die Vorrichtung je nach Anforderungen gesteuert werden, beispielsweise mit den Parametern Zeit, Luftstromintensität und verschiedener Geruchseindrücke (Anspruch 20).

Es kann vorgesehen werden, den Luftstrom der Einrichtung individuell regulierbar einzustellen, beispielsweise derart, daß das Gebläse mit einer Drehzahleinstelleinrichtung verbunden ist (Ansprüche 21 und 22).

In einer anderen Ausführung kann vorgesehen werden, daß das Duftstoffpräparat mit einem Duftstoffvorratsbehälter verbindbar ausgebildet ist, wodurch ein Auswechseln des Duftstoffpräparats entfällt (Anspruch 23).

Es ist vorteilhaft, das Duftstoffpräparat als einen saugfähigen Luftstromdiffusor bzw. Luftstromformungsvorsatz auszugestalten (Anspruch 24).

Durch die Erfindung ergeben sich folgende Vorteile:
- Die Duftstoffe werden nicht durch Wärmeentwicklung, sondern bei Raumtemperatur kalt aktiviert und freigesetzt (und sich dadurch im Geruchseindruck nicht durch die Wärme verändern).
- Die Duftstoffe werden durch eine natürliche Verdunstung von Alkohol in reproduzierbarer Menge freigesetzt und anschließend gezielt ausgerichtet.
- Die Duftstoffe breiten sich nicht gleichförmig im Raum aus, sondern werden zielgerichtet zur Nase der Ziel-Person geführt.
- Die Duftstoffe werden derart dosiert zur Nase geführt, daß die behandelte Person nicht in ihrem Gesichtsfeld eingeschränkt wird oder in möglicherweise die Haut reizenden Kontakt mit dem Duftstoff kommt.
- Die Atemluft wird derart mit Duft beaufschlagt, daß die Person frei beweglich ist und auch frei atmen kann, d.h. die Person muß nicht durch einen Luftfilter o.ä. hindurch atmen. Auch bei tiefen Atemzügen ist durch eine individuell festlegbare Konzentration der Duftstoffe keine Überdosierung möglich.
- Die Duftstoffe führen durch niedrigste Konzentration zu keinem Geruchsempfinden bzw. keiner Geruchsbelästigung anderer in demselben Raum anwesender Personen, d.h. die Wirkung ist ausschließlich auf die Zielperson beschränkt, eine Wirkung auf andere wird vermieden.
- In einer Ausführung mit einem Verwirbelungsgitter wird die Luftströmung so aufbereitet, daß für den Riecheffekt bezüglich des Einsaugens der Duftstoffe ideale Bedingungen durch feine Verwirbelungen und Bremsung der Luftströmung vorliegen, so daß die Konzentration der Duftstoffe sehr niedrig sein kann. Dadurch wird keine Inhalation der Duftstoffe ausgelöst, sondern gerade die Schwelle des Geruchsempfindens erreicht.
- Eine Bereitstellung der Duftstoffe im Nasenbereich ist beliebig an- und ausschaltbar, wodurch eine Geruchsblindheit der Nase verhindert werden kann.
- Die Duftart ist beliebig oft ohne einen Nebeneffekt eines Mischgeruchs austauschbar.
- Die Vorrichtung kann als eine Ergänzung bzw. als ein Zusatzangebot bei einem Haarbehandlungsgerät im Friseursalon vorgesehen werden.
- Eine Aktivierung des Dufteindrucks geschieht mit Hilfe einer Luftströmung, vorzugsweise mittels eines Ventilators.
- Eine Aktivierung des Dufteindrucks kann alternativ zu einem Ventilator auch mit einer alternativen Art der Strömungserzeugung geschehen, z. B. mit Hilfe von Druckluft oder Dampf, sofern diese Strömung gerichtet und regulierbar ist.
- Zu Beginn der Duftwirkung wird der Ventilator entweder eingeschaltet oder eine Abdeckung desselben entfernt.
- Besonders vorteilhaft ist es, wenn der Ventilator die auszustoßende Luft nicht aus dem möglicherweise mit anderen Gerüchen belasteten Behandlungsraum (des Friseurgeräts), sondern von außerhalb dieses Gebiets ansaugt.
- Bei einer Beduftung des Kunden während einer Dauerwellbehandlung kommt es nicht zu einer Verwirbelung der Luft und dadurch zu einem Transport der mit möglicherweise unangenehmen Gerüchen behafteten Behandlungsabluft in den Ansaugbereich der Nase. Dadurch wird das Abdampfen der Haarbehandlungsmittel, was besonders beim gleichzeitigen Einsatz von Wärme nach oben gerichtet ist, nicht beeinflußt.
- Durch die gerichtete Strömung in den Ansaugbereich der Nase überwiegt auch dann der angenehme Dufteindruck, wenn extrem leicht flüchtige Geruchsstoffe mit deutlich unangenehmem Geruch den auf dem Kopf bzw. den Haaren aufgebrachten kosmetischen Präparaten entweichen.
- Die Luftströmung des Ventilators kann auch während des Wirkungszeitraums zu- und abgeschaltet bzw. reguliert werden, wobei dies durch Abdecken der Luftströmung oder durch Ausschalten bzw. Regeln des Ventilators selbst geschehen kann. Die Regulierung des Luftstroms des Ventilators wird automatisch oder mit direkter Einflußnahme des Kunden vorgenommen.
- Eine entsprechende Regulierung kann durch die Vorrichtung gesteuert werden, die entsprechend an einem Gerät integriert ist oder in eine Programmsteuerung in der Vorrichtung selbst abgelegt ist. Bei einem automatischen Programmablauf wird der Programmstart der Duftvermittlung durch das Behandlungsgerät gestartet mittels einer Starttaste an der Vorrichtung oder durch Einstecken der Vorrichtung an ein Behandlungsgerät.
- Der Ventilator ist so dimensioniert und angeordnet, daß der Luftstrom gerade ausreicht, den von der Nase des Kunden einsaugbaren Luftbereich mit Duftstoffen zu versorgen, aber nicht den umgebenden Raum beduftet.
- Der Ventilator ist so positioniert bzw. derart mit einem Luftstromformungsvorsatz versehen, daß die Augen des Kunden nicht durch den Luftzug gereizt werden, wobei die Strömung z.B. bei Anordnung der Vorrichtung schräg vorne über dem Kunden in den Ansaugbereich der der Nase, also unterhalb derselben in Mund- bzw. Kinnhöhe geführt wird. Die Luftbewegung und Strömungsrichtung wird so eingestellt, daß die Augen der behandelten Person nicht gereizt werden, indem die Wirbelverhältnisse ein Ansaugen beim Atmen erfordern, um in direkten Kontakt mit dem Gesicht zu kommen.
- Es gibt verschiedene Ausführungsmöglichkeiten der Luftführung, wobei die Luftströmung so gerichtet sein kann, daß sie gleichzeitig eine angenehm kühlende Luftbewegung für die Kundin während der Wärmebehandlung des Kopfes erzeugt. Diese stellt aber nicht wie bei einem einfachen Lüfterbetrieb durch die definiert verlangsamte Luftgeschwindigkeit keinen Zug dar, sondern die angewärmte Luft außerhalb des eigentlichen Luftkegels des Lüfters wird durch den Luftsog mitgerissen und entsprechend bewegt, ohne einen unangenehmen kühlen Luftstrom zu bilden wie bei direkter Luftströmung in das Gesicht.
- In einer anderen Ausführung ist eine Strömung gerade soweit diffus vorgesehen, daß der Duft zwar noch in ausreichender Konzentration vor die Nase gelangt, nicht aber zu einer Zugempfindung führt. Für besonders zugempfindliche Personen kann die vom Lüfter angesaugte Luft etwas angewärmt sein, wobei hier darauf zu achten ist, daß die Lufttemperatur nicht zu einer negativen Veränderung des eingesetzten Duftes führt.
- Für den Kunden ist eine Drehzahleinstellung des Lüfters vorgesehen, über den sowohl die Intensität des Dufts als auch die Stärke des Luftstroms individuell angepaßt werden kann, In einer weiteren Ausführung kann die Intensität auch durch eine Verkleinerung der Luftstromaustrittsöffnung oder durch eine künstliche Verdichtung des Diffusors erreicht werden.
- In einer weiteren Ausführung sind zwei seitlich angeordnete Ventilatoren vorgesehen, die die mit Duftstoffen angereicherte Luft in den Bereich vor Kinn und Nase führen, wobei die Luftströmung derart geformt und gerichtet ist, daß keine Reizung der Ohren oder der Augen auftritt.
- Der Duft besteht aus mit einem leicht flüchtigem Stoff, vorzugsweise verdünntes Duftöl mit Alkohol.
- Das Duftstoffpräparat ist als Duftreservoir ausgebildet, das für die gesamte Zeit der Behandlung durch den Kapillareffekt ausreichend Duftstoff von innen nach außen nachliefert.
- Das Duftstoffpräparat ist vorzugsweise nicht in direktem Kontakt mit dem Lüfter bzw. den Strahlführungs- und Diffusorkomponenten, so daß diese nicht durch etwaige aggressive Duftstoffe angegriffen werden können.
- Das Duftstoffpräparat ist vorzugsweise als Verbrauchsteil (Einwegteil) ausgebildet, wobei für jede Beduftung ein neues, frisches und dadurch keimfreies Duftreservoir zum Einsatz kommt.
- In einer anderen Ausführung kann das Duftstoffpräparat aus einem saugfähigen Material (Vlies) bestehen und auch mit einem Vorratsbehälter verbunden sein, durch den bei Aktivierung eine Duftdosis freigesetzt wird und von dem Vlies aufgesaugt wird. Hierbei ist ein Verschluß des Vorratsbehälters wichtig, da eine permanente Nachbefeuchtung über einen gewissen Zeitraum doch zu einer Geruchsbelästigung im Anwendungs-Raum führen würde.
- Das Duftreservoir besteht aus einem an einem Träger befestigtem, mit dem Duft getränktem Streifen z.B. Viskosevlies, der in den Luftstrom nach Verlassen der Luftformungseinheit gebracht und dort fixiert wird.
- In einer anderen Ausführung ist der Luftstrom-Diffuser saugfähig ausgebildet und mit dem Duft benetzt und als Duftreservoir und Diffusor in einem wirkt. Hierbei kann sowohl das Material an sich saugfähig sein oder auch derart porös, daß sich der Duftstoff in ausreichender Menge in den Poren anlagert.

### Anwendungsbeispiele:

- Anwendung zur Verbesserung der Stimmung durch Auswahl eines angenehmen Geruchseindrucks.
- Anwendung zur Überbrückung von Wartezeiten, z. B. beim Friseur.
- Anwendung während der durch Wärmestrahlung unterstützten Einwirkung von unangenehm riechenden Haarbehandlungsmitteln, so daß der Duft die unangenehmen Geruchseindrücke ersetzt oder überlagert. Für diesen Zweck ist eine optimal Ausrichtung der Luftbewegung notwendig, so daß die aufsteigenden Kosmetikdämpfe nicht durch die Duft-Strömung verwirbelt werden und in Nasennähe kommen. Einsatzgebiete sind z.B. Dauerwelle, Haarfärbung, Haartönung, Haartrocknung und Einwirkung von Pflegemitteln.

Die Erfindung wird anhand mehrerer Figuren näher beschrieben.

Es zeigt:
- Fig. 1: eine Vorrichtung zur Vermittlung eines bestimmten Geruchseindrucks auf eine Person;
- Fig. 2 und 3: die Vorrichtung in verschiedenen Ansichten mit einem ersten Duftstoffpräparat;
- Fig. 4: eine erste Halteeinrichtung für ein Duftstoffpräparat;
- Fig. 5: die Vorrichtung mit einer zweiten Halteeinrichtung für ein Duftstoffpräparat;
- Fig. 6: die zweite Halteeinrichtung für ein Duftstoffpräparat in Perspektive;
- Fig. 7: in einer Seitenansicht die Vorrichtung in Verwendung mit einem Haarbehandlungsgerät, und
- Fig. 8: die Vorrichtung nach der Fig. 7 in einer Vorderansicht.

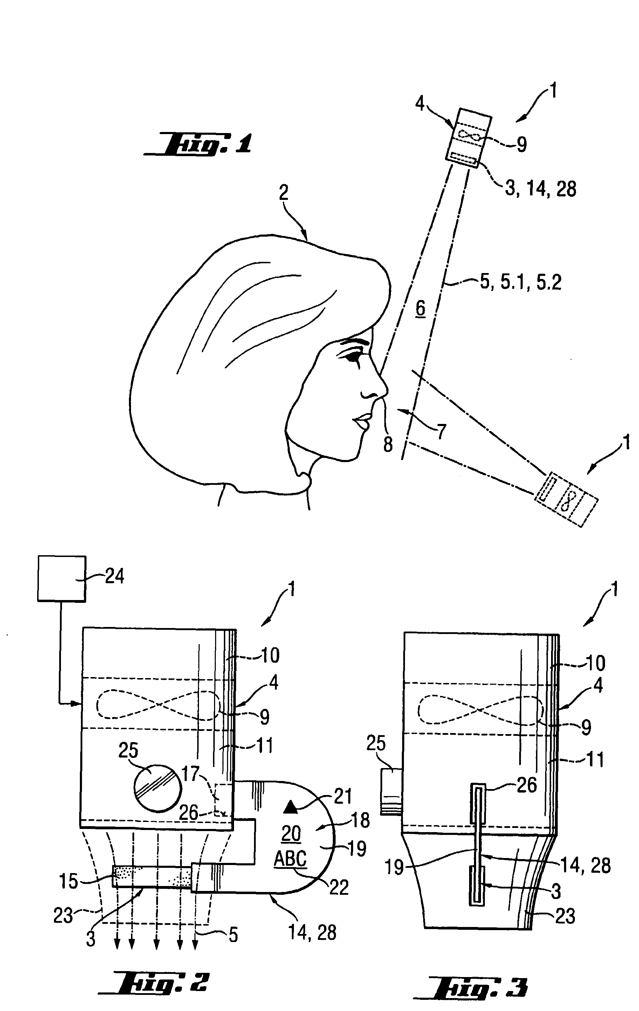

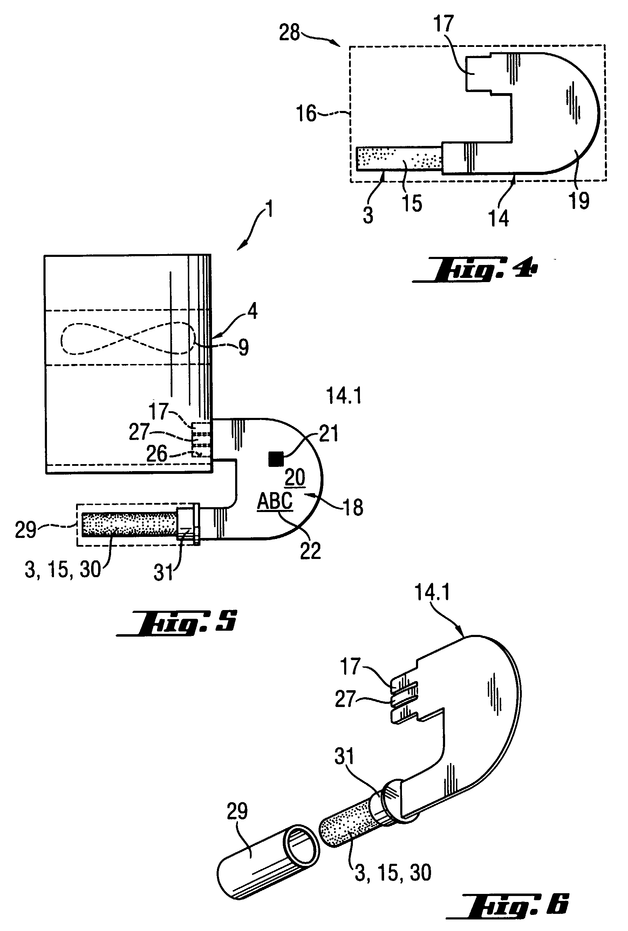

Fig. 1 zeigt eine Vorrichtung 1 zur Vermittlung eines bestimmten Geruchseindrucks auf eine Person 2 mittels eines Duftstoffpräparats 3. Die Vorrichtung 1 ist mit einer Einrichtung 4 zur Erzeugung mindestens eines gebündelten Luftstromes 5 versehen, wobei das Duftstoffpräparat 3 innerhalb des Luftstromes 5 angeordnet ist und vom Luftstrom 5 umspült oder/und durchspült wird. Der so mit einem Duft 6 versehene Luftstrom 5 ist wenigstens ungefähr auf einen Ansaugbereich 7 einer Nase 8 der Person 2 gerichtet. Die Vorrichtung 1 kann beliebig - je nach Anforderungen - in Winkelstellung wie auch in Entfernung zur Nase 8 mittels dazu geeigneter Mittel positioniert (wie gestrichelt angedeutet) und wahlweise mit einem Gerät 12 (Fig. 7 und 8) kombiniert werden. So ist es zum Beispiel während einer Haarbehandlung mit dem Gerät 12 möglich, zusätzlich ein angenehmes Geruchserlebnis zu vermitteln. Dadurch werden gegebenenfalls auch (unangenehme) Außengerüche eleminiert. Die Einrichtung 4 ist mit mindestens einem elektrischen Gebläse 9 versehen. Es kann aber auch wahlweise anstelle des Gebläses 9 Druckluft 5.1 aus einem nicht dargestellten Druckluftspeicher oder Dampf 5.2 aus einer nicht dargestellten Dampfquelle vorgesehen werden.

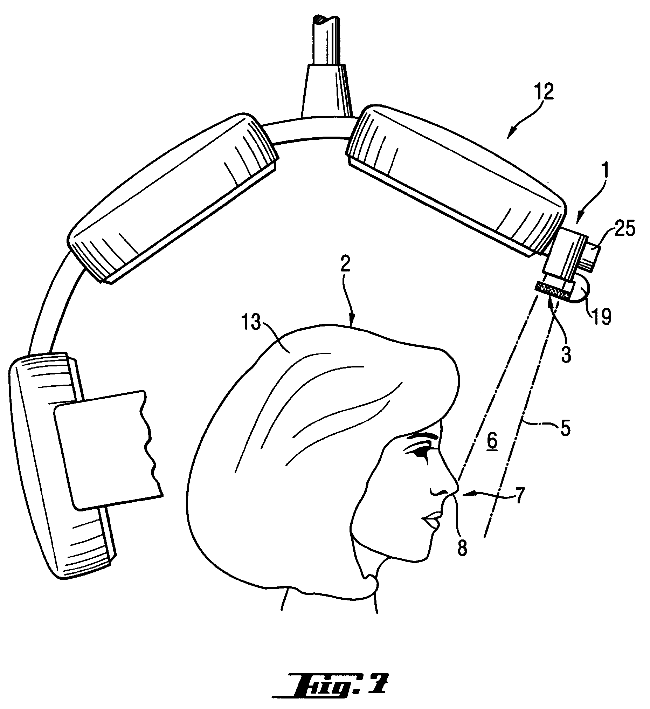

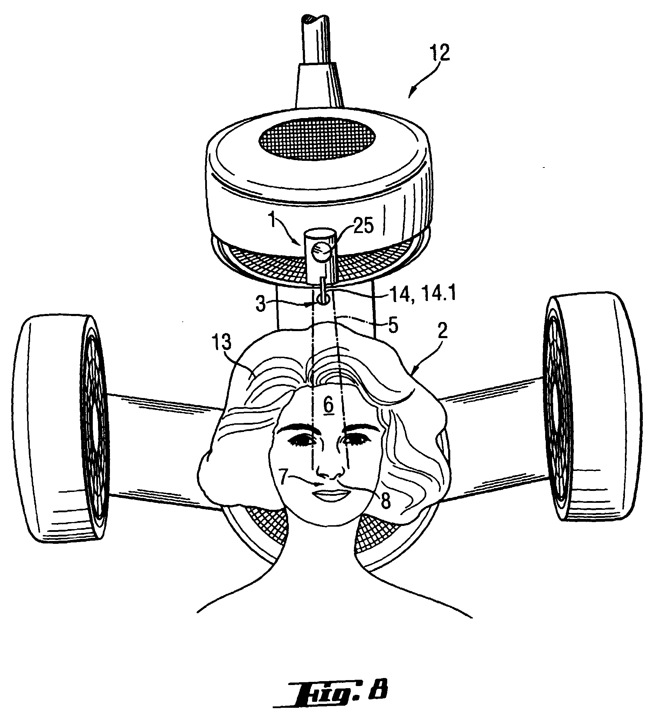

Aus den Fig. 2 und 3 geht die Vorrichtung 1 näher hervor. So ist an einem Ende der Vorrichtung 1 ein Luftansaugteil 10, dann das elektrische Gebläse 9 und am anderen Ende ein Luftströmungsgleichrichter 11 angeordnet. Der aus dem Luftströmungsgleichrichter 11 austretende Luftstrom 5 umspült oder/und durchspült das Duftstoffpräparat 3, das auf einer Karte 14 aufgebracht ist.

Das Duftstoffpräparat 3 ist auf einer Halteeinrichtung 19 aufgebracht. Zum Verbinden der Halteeinrichtung 19 an der Vorrichtung 1 ist dieses mit einem Steckverbindungsteil 17 versehen, das mit einer entsprechenden Aufnahme 26 der Vorrichtung 1 verbunden ist. Die Oberfläche der Halteeinrichtung 19 ist mit einer Information 18 über die Duftstoffart versehen, beispielsweise mittels einer bestimmten Farbgebung 20 oder/und eines bestimmten Symbols 21 oder/und eines Beschreibungsaufdrucks 22. Durch einen gestrichelt dargestellten, düsenartigen Luftstromformungsvorsatz 23 an der Vorrichtung 1 wird die Luftströmung 5 so aufbereitet, daß für einen Riecheffekt bezüglich eines Einsaugens von Duftstoffen mittels einer Nase 8 ideale Bedingungen durch feine Verwirbelungen und Bremsung der Luftströmung vorliegen, so daß eine niedrige Konzentration von Duftstoffen ausreicht. Dadurch wird keine Inhalation von Duftstoffen ausgelöst, sondern gerade die Schwelle des Geruchsempfindens erreicht. Mittels einer schematisch dargestellten Programmsteuerung 24 kann die Vorrichtung 4 je nach Anforderung gesteuert werden, zum Beispiel mit den Parametern Zeit, Luftstromintensität und Wechseln der Duftstoffart durch Wechseln der Duftstoffpräparate 3. Durch eine Drehzahleinstelleinrichtung 25 des Gebläses 9 läßt sich der Luftstrom 5 individuell einstellen.

Die Fig. 4 zeigt das auf einer Karte 14 aufgebrachte Duftstoffpräparat 3 und ist als Einwegteil 28 mit einer Duftschutz-Umhüllung 16 versehen, die vor Benutzung des Duftstoffpräparats 3 entfernt wird.

In der Fig. 5 ist die Vorrichtung 1 mit einem zylinderförmigen Duftstoffpräparatträger 30 dargestellt, welcher vor Gebrauch mit einer Umhüllungskappe 29 verschlossen ist und dadurch auch für eine Duftprobe kurzzeitig abgenommen werden kann. Für eine sichere Verbindung mit der Aufnahme 26 ist das Steckverbindungsteil 17 mit einer Federzunge 27 (Fig. 6) versehen, die sich in der Aufnahme 26 wieder lösbar verrastet. Die Karte 14.1 ist mit dem Steckverbindungsteil 17 und einem Aufnahmeteil 31 für den zylinderförmigen Duftstoffpräparatträger 30 einstückig aus Kunststoff hergestellt.

In den Fig. 7 und 8 ist die Vorrichtung 1 am Beispiel eines Wärmebehandlungsgeräts 12 für Kopfhaar, wie es von der Anmelderin unter der Bezeichnung 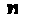 Climazon Millennium" vertrieben wird, dargestellt.

### Bezugsziffernliste

- 1: Vorrichtung
- 2: Person
- 3: Duftstoffpräparat
- 4: Einrichtung/Luftstromerzeugung
- 5: Luftstrom
- 5.1: Druckluft
- 5.2: Dampf
- 6: Duft
- 7: Ansaugbereich
- 8: Nase
- 9: Gebläse
- 10: Luftansaugteil
- 11: Luftströmungsgleichrichter
- 12: Gerät
- 13: Kopfhaar
- 14,14.1: Karte
- 15: Saugfähiges Material
- 16: Umhüllung
- 17: Steckverbindungsteil
- 18: Information
- 19: Halteeinrichtung
- 20: Farbgebung
- 21: Symbol
- 22: Beschreibungsaufdruck
- 23: Luftstromformungsvorsatz
- 24: Programmsteuerung
- 25: Drehzahleinstelleinrichtung
- 26: Aufnahme
- 27: Federzunge
- 28: Einwegteil
- 29: Umhüllungskappe (hohlzylindrisch)
- 30: Zylinderförmiger Duftstoffpräparatträger
- 31: Aufnahmeteil

## Patentansprüche

1. Verfahren zur Vermittlung eines bestimmten Geruchseindrucks auf eine Person (2) mittels eines Duftstoffpräparats (3),
**dadurch gekennzeichnet ,**
daß ein gebündelter Luftstrom (5) wenigstens ungefähr auf einen Ansaugbereich (7) einer Nase (8) der Person (2) gerichtet wird, wobei das Duftstoffpräparat (3) vom Luftstrom (5) umspült oder/und durchspült wird.

2. Vorrichtung (1) zur Vermittlung eines bestimmtem Geruchseindrucks auf eine Person (2) mittels eines Duftstoffpräparats (3), **dadurch gekennzeichnet**, daß die Vorrichtung (1) mit einer Einrichtung (4) zur Erzeugung mindestens eines gebündelten Luftstromes (5) versehen ist, wobei das Duftstoffpräparat (3) innerhalb des Luftstromes (5) angeordnet ist und vom Luftstrom (5) umspült oder/und durchspült wird, und daß der so mit einem Duft (6) versehene Luftstrom (5) wenigstens ungefähr auf einen Ansaugbereich (7) einer Nase (8) der Person (2) gerichtet ist.

3. Verfahren nach Anspruch 1 oder Vorrichtung nach Anspruch 2, dadurch gekennzeichnet, daß der Luftstrom (5) ungefähr eine Raumtemperatur aufweist.

4. Vorrichtung nach Anspruch 2, dadurch gekennzeichnet, daß die Einrichtung (4) mit mindestens einem elektrischen Gebläse (9) versehen ist.

5. Vorrichtung nach Anspruch 2, dadurch gekennzeichnet, daß dem Gebläse (9) ein Luftansaugteil (10), ein Luftströmungsgleichrichter (11) und das Duftstoffpräparat (3) zugeordnet ist.

6. Vorrichtung nach Anspruch 2, dadurch gekennzeichnet, daß die Vorrichtung (1) mit einem Gerät (12) zum Behandeln von Kopfhaar (13) verbunden ist.

7. Vorrichtung nach mindestens Anspruch 2, dadurch gekennzeichnet, daß das Duftstoffpräparat (3) mit einer Halteeinrichtung (19) versehen ist.

8. Vorrichtung nach Anspruch 7, dadurch gekennzeichnet, daß das Duftstoffpräparat (3) und die Halteeinrichtung (19) als eine Karte (14) ausgestaltet ist.

9. Vorrichtung nach Anspruch 2, dadurch gekennzeichnet, daß das Duftstoffpräparat (3) von einem saugfähigem Material (15) aufgenommen ist.

10. Vorrichtung nach Anspruch 9, dadurch gekennzeichnet, daß das saugfähige Material (15) als eine Karte (14) ausgestaltet ist.

11. Vorrichtung nach mindestens Anspruch 8, dadurch gekennzeichnet, daß die Karte (14,14.1) mit einer Duftschutz-Umhüllung (16) versehen ist.

12. Vorrichtung nach Anspruch 9, dadurch gekennzeichnet, daß das saugfähige Material (15) als ein Zylinder (30) ausgestaltet ist.

13. Vorrichtung nach Anspruch 12, dadurch gekennzeichnet, daß der Zylinder (30) mit einer Umhüllungskappe (29) versehen ist.

14. Vorrichtung nach mindestens Anspruch 8, dadurch gekennzeichnet, daß ein System mit unterschiedlichen Duftstoffarten vorgesehen ist.

15. Vorrichtung nach mindestens Anspruch 8, dadurch gekennzeichnet, daß das Duftstoffpräparat (3) mit der Halteeinrichtung (19) als Einwegteil (28) vorgesehen ist.

16. Vorrichtung nach Anspruch 14, dadurch gekennzeichnet, daß die Halterichtung (19) mit einer Information (18) über die Duftstoffart versehen ist.

17. Vorrichtung nach Anspruch 15, dadurch gekennzeichnet, daß als Information (18) eine bestimmte Farbgebung (20) oder/und ein bestimmtes Symbol (21) oder/und ein Beschreibungsaufdruck (22) vorgesehen ist.

18. Vorrichtung nach Anspruch 2, dadurch gekennzeichnet, daß die Einrichtung (4) mit einem Luftstromformungsvorsatz (23) versehen ist.

19. Vorrichtung nach Anspruch 2, dadurch gekennzeichnet, daß der Luftstrom (5) von Druckluft (5.1) oder Dampf (5.2) gebildet ist.

20. Vorrichtung nach Anspruch 6, dadurch gekennzeichnet, daß die Vorrichtung (1) von einer Programmsteuerung (24) gesteuert ist.

21. Vorrichtung nach Anspruch 2, dadurch gekennzeichnet, daß der Luftstrom (5) der Einrichtung (4) regulierbar ist.

22. Vorrichtung nach Anspruch 4, dadurch gekennzeichnet, daß das Gebläse (9) mit einer Drehzahleinstelleinrichtung (25) verbunden ist.

23. Vorrichtung nach Anspruch 2, dadurch gekennzeichnet, daß das Duftstoffpräparat (3) mit einem Duftstoffvorratsbehälter verbindbar ausgebildet ist.

24. Vorrichtung nach Anspruch 2, dadurch gekennzeichnet, daß das Duftstoffpräparat (3) als ein saugfähiger Luftstromdiffusor ausgebildet ist.
